# EUROPEAN PATENT APPLICATION

(11) **EP 4 674 426 A1**
(43) Date of publication of application: **07.01.2026**
(21) Application number: 24762992.6
(22) Date of filing: 18.02.2024
(51) Int. Cl.: A61K 38/17, A61P 17/14, A61M 37/00

(54) **USE OF ANNEXIN A5 IN HAIR GROWTH, HAIR NURTURING, HAIR CARE, HAIR LOSS PREVENTION, AND HAIR LOSS TREATMENT, AND RELATED PRODUCTS AND PHARMACEUTICAL COMPOSITIONS**

(30) Priority: 27.02.2023 CN 202310170435; 13.11.2023 CN 202311506076
(71) Applicant: Shanghai Celleaf Biotechnology Co., Ltd., Shanghai 200241 (CN)
(72) Inventor: ZHANG, Wenjie, Shanghai 200241 (CN); YU, Jiaojiao, Shanghai 200241 (CN)
(74) Representative: Mewburn Ellis LLP
(86) International application number: PCT/CN2024/077422
(87) International publication number: WO 2024/179309

(57) **Abstract**

A use of annexin A5 in hair growth, hair nurturing, hair care, hair loss prevention, and hair loss treatment, and related products and pharmaceutical compositions. The use of annexin A5 includes hair growth, hair nurturing, hair care, hair loss prevention, and/or hair loss treatment. Annexin A5 can effectively promote hair regeneration after androgenic alopecia, and can be used as an active ingredient in products for hair growth, hair nurturing, hair care, and hair loss prevention and pharmaceutical compositions for hair loss treatment.

## Description

### Technical field

The present invention belongs to the field of pharmaceutical biotechnology, and specifically relates to the use of Annexin A5 in hair growth, hair nurturing, hair care, hair loss prevention, and hair loss treatment, and related products.

### Background

Hair loss is a common clinical condition, and its etiology has not been fully elucidated. Studies have shown that it may be associated with factors such as age, immune function, genetics, hormonal levels, local inflammatory responses, neurological factors, and environmental factors. At present, the common types of hair loss mainly include androgenetic alopecia, alopecia areata, Telogen effluvium, senescent alopecia, etc. Among these, androgenetic alopecia is the most prevalent, primarily affecting men, and exhibits a strong hereditary predisposition. At present, the only two androgenetic alopecia treatments approved by the U.S. FDA are finasteride and minoxidil, yet patients often experience significant adverse reactions or side effects when using these medications. Therefore, there is an urgent need in this field to develop a new product for hair growth, hair nurturing, hair care, and hair prevention, as well as a drug for treating hair loss with no adverse reactions or side effects on the human body.

### Summary of the invention

The purpose of the present invention is to provide a new product for hair growth, hair nurturing, hair care, and hair prevention, as well as a drug for treating hair loss with no adverse reactions or side effects on the human body.

In the first aspect of the present invention, provided is a use of Annexin A5 in the preparation of a pharmaceutical composition or a medical device for treating hair loss.

In another preferred embodiment, the hair loss is selected from the group consisting of: androgenetic alopecia, alopecia areata, Telogen effluvium, senescent alopecia, psychogenic alopecia, physical alopecia, chemical alopecia, nutritional alopecia, infectious alopecia, and endocrine alopecia.

In another preferred embodiment, the pharmaceutical composition is used for (1) promoting hair growth; (2) regenerating hair after hair loss; (3) improving the proliferation ability of cells in hair follicles; (4) increasing the number of capillaries around dermal papillae; and (5) inhibiting the expression of androgen receptor (AR) and transforming growth factor β1 (TGF - β 1) in hair follicles.

In another preferred embodiment, the pharmaceutical composition is used for (1) inhibiting the expression of TGF - β1, IL-6, THF - α, and IL-1 β in the skin; (2) reducing the content of intracellular dihydrotestosterone (DHT); and/or (3) enhancing the in *vitro* growth ability of human dermal papilla cells.

In another preferred embodiment, the content of dihydrotestosterone is dihydrotestosterone in dermal papilla cells.

In another preferred embodiment, the dermal papilla cells are human primary dermal papilla cells.

In the second aspect of the present invention, provided is a use of Annexin A5 in the preparation of a product for treating hair growth and/or preventing hair loss.

In another preferred embodiment, the product is used for (a) improving the smoothness of hair; (b) reducing hair loss; (c) increasing hair growth density; and/or (d) promoting hair growth.

In another preferred embodiment, the product is used for hair nurturing and/or hair care.

In the third aspect of the present invention, provided is a pharmaceutical composition for treating hair loss, comprising: (i) Annexin A5; (ii) other drugs for treating hair loss; and (iii) pharmaceutically acceptable carriers.

In another preferred embodiment, the carrier is selected from the group consisting of: diluents, excipients, fillers, binders, wetting agents, disintegrants, absorption enhancers, surfactants, adsorption carriers, and lubricants.

In another preferred embodiment, the dosage form of the pharmaceutical composition is selected from the group consisting of: liniments, sprays, lotions, ointments, and injections.

In another preferred embodiment, the pharmaceutical composition is introduced into the body tissue by a method selected from the group consisting of: injection, spray, permeation, absorption, physically mediated method, and chemically mediated method, or by mixed with or encapsulated by other substances before being introduced into the body tissue.

In another preferred embodiment, the other drugs are selected from the group consisting of: Qingwanye, minoxidil, and finasteride.

In the fourth aspect of the present invention, provided is a method for reducing intracellular DHT concentration in vitro, comprising a step of culturing cells in the presence of Annexin A5, thereby reducing intracellular DHT concentration.

In another preferred embodiment, the method is non-therapeutic and non-diagnostic.

In another preferred embodiment, the cells are human dermal papilla cells.

In another preferred embodiment, the Annexin A5 is exogenously added.

In another preferred embodiment, the concentration of Annexin A5 is 1-1000ng/ml.

In another preferred embodiment, the method further comprises a step of detecting the concentration of intracellular DHT and comparing it with the concentration of intracellular DHT before the addition of Annexin A5.

In the fifth aspect of the present invention, provided is a method for enhancing the growth ability of dermal papilla cells in vitro, comprising a step of culturing dermal papilla cells in the presence of Annexin A5, thereby enhancing the growth ability of the dermal papilla cells.

In another preferred embodiment, the method is non-therapeutic and non-diagnostic.

In another preferred embodiment, the cells are human dermal papilla cells.

In another preferred embodiment, the Annexin A5 is exogenously added.

In another preferred embodiment, the concentration of Annexin A5 is 1-1000ng/ml.

It should be understood that within the scope of the present invention, the above-described technical features of the present invention and the technical features described in detail below (e.g., examples) may be combined with each other to constitute a new or preferred technical solution. Limited by space, it will not be repeated here.

### Drawings

Figure 1 shows the therapeutic effect of Annexin A5 on androgenetic alopecia in mice;
Figure 2 shows the histological HE staining results after treatment with Annexin A5 for androgenetic alopecia;
Figure 3 shows the immunohistochemical staining results after treatment with Annexin A5 for androgenetic alopecia;
Figure 4 shows the results of expression of androgen receptor and TGF-β1 in mouse hair follicle specimens after treatment with Annexin A5 for androgenetic alopecia.

### DETAILED DESCRIPTION OF THE INVENTION

After extensive and in-depth research, through extensive screening, the inventor has developed for the first time a novel use of annexin A5 in hair growth, hair nurturing, hair care, hair loss prevention, and hair loss treatment, and related products and pharmaceutical compositions. The experimental results effectively confirmed that Annexin A5 can enhance the proliferation ability of cells in the hair follicles of the androgenetic alopecia model mice, promote the number of capillaries around the dermal papillae, and inhibit the expression levels of AR and TGF-β1 in the dermal papillae of the mice, which further promotes hair regenerative ability after androgenetic alopecia. Therefore, Annexin A5 can be used in hair growth, hair nurturing, hair care, hair loss prevention, and hair loss treatment. On this basis, the present invention has been completed.

### Annexin A5

Annexin A5 (AnxA5) is a calcium phospholipid binding protein composed of a single polypeptide chain of 319 amino acids, and is an important functional protein molecule in the human body, widely distributed in cells and body fluids. Annexin A5 is a member of the Annexin family, with a specific structure and unique biochemical characteristics. It can reversibly, specifically, and efficiently bind to phosphatidylserine molecules in a calcium ion dependent manner. Based on this characteristic, Annexin A5 plays an important biological function and plays a crucial role in numerous physiological and pathological processes in the human body. As early as 1985, researchers first proposed that Annexin A5 possesses anticoagulant activity and is also known as an anticoagulant protein. In recent years' research, with the deepening of related research, Annexin A5 also involves in various functions such as inflammatory response, apoptosis process, and regulation of tumor development. At present, there is no relevant research revealing the application of Annexin A5 in hair growth, hair nurturing, hair care, and hair loss treatment.

### Pharmaceutical compositions and administration

Annexin A5 can be prepared as pharmaceutical compositions in dosage forms such as tablets, capsules, powders, microgranules, solutions, lozenges, jellies, creams, spirits, suspensions, tinctures, poultices, liniment, lotions, injections and aerosols, etc. Pharmaceutical compositions can be prepared by commonly known preparation techniques, and suitable pharmaceutical additives can be added to the drug.

Examples of pharmaceutical excipients include pharmaceutically acceptable carriers, excipients, diluents, binders, decomposing agents, fillers, binders, wetting agents, lubricants, flow aids, disintegrants, absorption enhancers, surfactants, suspending agent, emulsifiers, stabilizers, insulating (wetting) agents, preservatives, solvents, solubilizers, preservatives, flavorings, sweeteners, dyes, fragrances, propellants, adsorption carriers, etc. These pharmaceutical excipients can be selected and added in appropriate amounts within the scope that does not affect the effectiveness of the present invention.

"Pharmaceutically acceptable carrier" refers to one or more compatible solid or liquid filler or gel substances that are suitable for human use and must have sufficient purity and sufficiently low toxicity. "Compatibility" herein refers to the ability of the components of a composition to blend with Annexin A5 and with each other without significantly reducing the efficacy of Annexin A5. Examples of pharmaceutically acceptable carrier include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as Tween^{®}), wetting agents (such as sodium dodecyl sulfate), colorants, flavoring agents, stabilizers, antioxidants, preservatives, pyrogen-free water, etc.

The administration modes of the pharmaceutical compositions of the present invention are not particularly limited, and representative modes of administration include (but are not limited to) oral, rectal, parenteral (intravenous, intramuscular or subcutaneous), and topical administration.

In addition to these inert diluents, the compositions may also contain auxiliaries such as wetting agents, emulsifiers, and suspending agents, sweeteners, flavoring agents and spices.

The suspensions may comprise suspending agents, such as ethoxylated isooctadecanol, polyoxyethylene sorbitol and dehydrated sorbitol esters, microcrystalline cellulose, methanolic aluminum, agar, and the mixtures thereof.

The compositions for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof

The dosage forms of Annexin A5 for topical administration include liniments, sprays, lotions, ointments, unguents, powders, patches, propellants, and inhalants. Annexin A5 is mixed with physiologically acceptable carriers and any preservatives, buffers or propellants that may be required if necessary under sterile conditions.

Annexin A5 can be administered alone, or in combination with other pharmaceutically acceptable drugs used for treating hair loss.

### Products containing Annexin A5

Annexin A5 described in the present invention can be prepared into various dosage forms, such as emulsions, liquids, ointments, creams, pastes, cakes, powders, and the like.

Within the scope that not hindering the effect of the present invention, other components that are general physiologically acceptable can be added to the products of the present invention, such as film forming agents, oil soluble gelling agents, organic modified clay minerals, resins, humectants, preservatives, antibacterial agents, fragrances, salts, antioxidants, pH adjusters, chelating agents, cooling agents, anti-inflammatory agents, vitamins, amino acids, nucleic acids, hormones, inclusion compound, etc.

Oil soluble gelling agents are selected from aluminum stearate, magnesium stearate, zinc myristate and other metal soaps; N-lauroyl-L-glutamic acid, α, γ - di-n-butylamine and other amino acid derivatives; cyclodextrin fatty acid esters such as cyclodextrin palmitate, cyclodextrin stearate, and cyclodextrin 2-ethylhexanoate palmitate; sucrose fatty acid esters such as sucrose palmitate and sucrose stearate; sorbitan benzylidene derivatives such as monobenzylidene sorbitol and di benzylidene sorbitol; organically modified clay minerals such as dimethylbenzyl dodecyl ammonium montmorillonite clay, dimethyloctacosanyl ammonium montmorillonite clay. One, or two or more of the above may be used as needed.

Humectants include: glycerol, sorbitol, propylene glycol, dipropylene glycol, 1,3-butanediol, glucose, xylitol, maltitol, polyethylene glycol, hyaluronic acid, chondroitin sulfate, pyrrolidone carboxylate, polyoxyethylene methyl glucoside, Polyoxypropylene methyl glucoside , etc.

Antibacterial preservatives include alkyl parabens, benzoic acid, sodium benzoate, sorbic acid, potassium sorbate, phenoxyethanol, etc. Antibacterial agents include benzoic acid, salicylic acid, carbolic acid, sorbic acid, alkyl parabens, p-chloro-m-cresol, hexachlorophene, benzalkonium chloride, chlorhexidine chloride, triclocarban, triclosan, photopigment, phenoxyethanol, etc.

Antioxidants include tocopherol, butylated hydroxyanisole, butylated hydroxytoluene, phytic acid, etc. pH adjusters include lactic acid, citric acid, glycolic acid, succinic acid, tartaric acid, dl-malic acid, potassium carbonate, sodium bicarbonate, ammonium bicarbonate, etc. Chelating agents include alanine, sodium ethylenediaminetetraacetate, sodium polyphosphate, sodium metaphosphate, phosphoric acid, etc. Cooling agents include L-menthol, camphor, etc. Anti-inflammatory agents include allantoin, glycyrrhetinic acid, glycyrrhizic acid, tranexamic acid, Azulene, etc.

Amino acids include glycine, valine, leucine, isoleucine, serine, threonine, phenylalanine, arginine, lysine, aspartic acid, glutamic acid, cystine , cysteine, methionine, tryptophan, etc. Nucleic acids include deoxyribonucleic acid, and hormones include estradiol, ethenyl estradiol, etc.

There is no special limit on the form of products, which can be liquid, lotion, frost, solid, paste, gel, powder, multi-layer, mousse, spray, etc.

### The main advantages of the present invention include:

(1) The present invention discloses for the first time the use of Annexin A5 in hair growth, hair nurturing, hair care, hair loss prevention, and hair loss treatment.
(2) Annexin A5 disclosed in the present invention can be applied topically by injection or application, and is easy to use.
(3) Annexin A5 disclosed in the present invention effectively enhances the proliferation of cells in the hair follicles of androgenetic alopecia model mice, promotes the number of capillaries around the dermal papillae, and inhibits the expression levels of AR and TGF-β1 in the dermal papillae of mice.
(4) Annexin A5 disclosed in the present invention can effectively improve hair regenerative ability after androgenetic alopecia, and can be effectively used as an active ingredient in products for hair growth, hair nurturing, hair care, hair loss prevention, and in pharmaceutical compositions for hair loss treatment.

The present invention will be further explained below in combination with specific examples. It should be understood that these examples are only used to illustrate the present invention and not to limit the scope of the present invention. In the following examples, the test methods without specific conditions are usually in accordance with conventional conditions or conditions recommended by the manufacturer. Unless otherwise specified, percentages and parts are calculated by weight.

The experimental materials and reagents used in the following examples are commercially available unless otherwise specified.

### Example 1: Establishment of a Mouse androgenetic Hair Loss Model and Preparation of Tissue section

Experimental animals: C57BL/6 male mice, 5-7 weeks old, weighing around 20g were housed in a 12 hour light/12 hour dark environment, with free access to food and water. The experiment set up control group (CTRL); model group (DHT); A5-Low group (treated with Annexin A5, injected once every three days); and A5-High group (treated with Annexin A5, injected once a day). Each group consists of 6 mice.

Modeling and administration: Dihydrotestosterone (DHT) was used to induce the formation of the mouse androgenetic hair loss model. In addition to the control group, the other three groups of mice received subcutaneous injection of DHT solution (5mg/mL) at the neck, 0.01mL for each, for a total of 2 weeks. The control group was injected with an equal volume of physiological saline. After 2 weeks, all mice were shaved and their back hair was removed using a shaving knife. Then hair removal cream was used to further remove residual hair from the shaved area of the mice. After hair removal, both the control group and the model group were injected daily with 2mL of PBS in the hair removal area, divided into 10 injection points, with 20 µ L per point. The A5-Low group received Annexin A5 treatment once every three days, while the A5-High group received Annexin A5 treatment once a day. The injection method was subcutaneous injection at 10 points in the hair removal area, with 20 µ L of Annexin A5 at a concentration of 50 µ g/mL injected at each point. The status of hair growth on the back of mice was observed every day and recorded by taking photos. Samples were collected after 16 days.

Tissue sampling: After drug treatment, mice were euthanized by cervical dislocation. In the new hair growth area of mouse, a shaver and a scissor were used to remove the mouse hair as much as possible, and then a small piece of skin tissue with a size of about 0.5cm x 0.5cm from the center of the depilation area was taken, and soaked in 4% paraformaldehyde and fixed for 24 hours. The ratio of the volume of paraformaldehyde used for fixing to the volume of tissue was approximately 4:1.

Preparation of tissue sections: After the skin tissue was fixed, different concentrations of ethanol were used for tissue dehydration. After dehydration, anhydrous ethanol and xylene were used as clearing reagent to clear the tissue. After clearing, the tissue was placed in paraffin for wax immersion treatment. After that, the tissue was placed in the heating block of the embedding machine to melt, and a mold was used to embed the wax block. Sections with a thickness of 5 µm was cut out using a paraffin tissue slicer and placed in a tissue cassette and then placed in a 60 °C drying box for 24 hours.

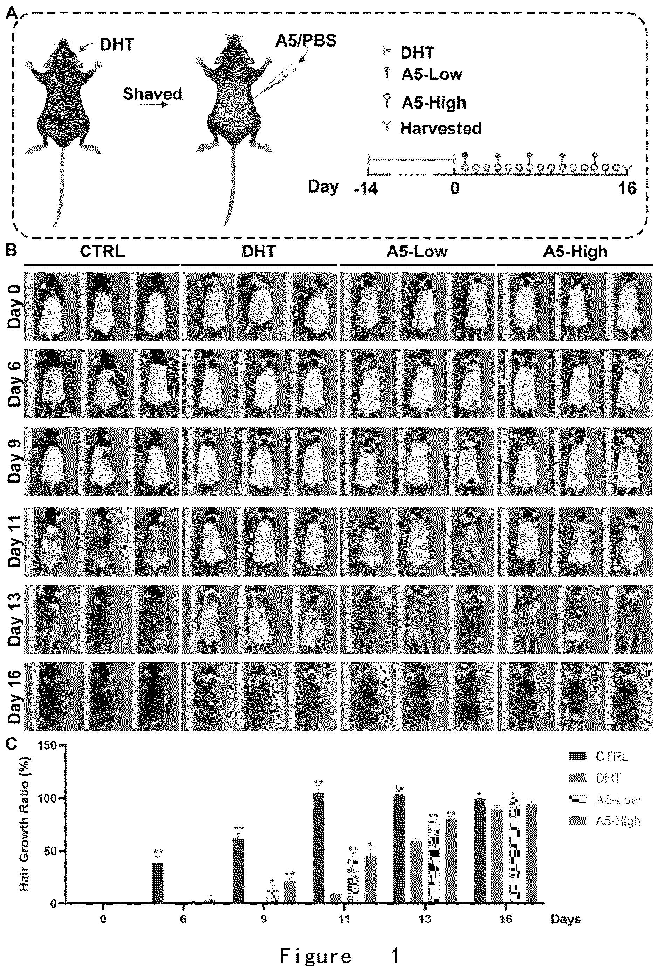

Results: As shown in Figure 1, by observing the hair growth of mice, it can be seen that the mice of the control group showed partial black skin in the depilated area on the 6th day, and by the 11th day, the skin of the mice had basically turned black. However, the black skin of DHT injected mice appeared later, that is, the mice entered the anagen phase more slowly. Compared with the control group, mice treated with Annexin A5 exhibited significantly enhanced hair growth ability, and both entered the anagen phase earlier, indicating that Annexin A5 effectively promoted hair growth in mice.

### Example 2: Histological HE staining analysis

Sections were taken for xylene dewaxing and gradient ethanol hydration, and then hematoxylin and eosin staining was performed. After staining, dehydration and clearing treatment were performed again. After treatment, the tissue was added dropwise with neutral resin, covered with a cover glass, and left overnight at room temperature. The next day, an optical microscope was used to observe and record, and the number of hair follicles was statistical analyzed.

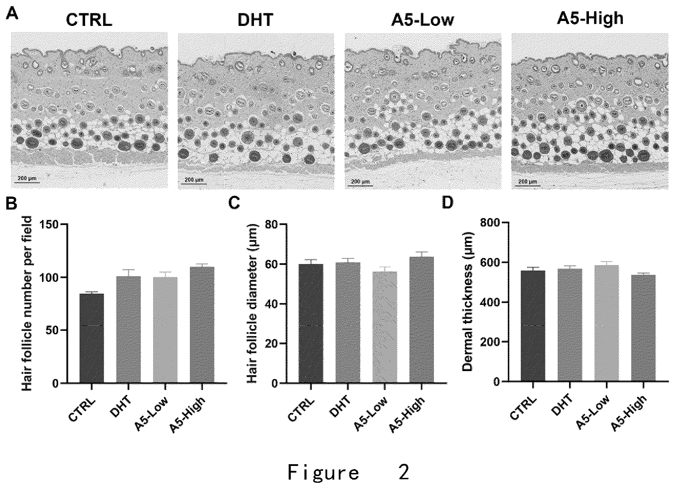

Results: As shown in Figure 2, through histological HE staining observation, it was found that DHT modeling injection and treatment with Annexin A5 maintained for 2 weeks did not affect the number of hair follicle dermal papillae, follicle size, or dermal thickness in mice.

### Example 3: Immunohistochemical analysis of paraffin sections

Sections were taken for xylene dewaxing and gradient ethanol hydration to block endogenous peroxidase, and sections were prepared for heat induced epitope retrieval. Then, 0.2% TritonX-100 solution was used for perforation treatment. After treatment, the section was drawn a circle around the tissue (to prevent antibody leakage) using a PAP pen, added dropwise with 10% normal rabbit serum (or other serum) to the circle to evenly cover the tissue, and incubated in a 37 °C oven for 30 minutes for blocking. The blocking solution was gently shaken off. CD31 (vascular angiogenesis marker) and Ki67 (cell proliferation marker) antibodies were prepared at a dilution ratio of 1:400, and dropped onto the sections respectively, and incubation was performed overnight at 4 °C. On the next day, the section was washed three times with PBS (pH 7.4), spun dry slightly, added with secondary antibody in the circle to cover the tissue, and incubated at 37 °C for 45 minutes. Subsequently, DAB color developmemt reaction was performed, and the color development time was controlled under a microscope. Brownish yellow was referred to as positive results, and the sections were washed with distilled water to terminate color development. After color development, the cell nucleus was counterstained with hematoxylin, washed, dehydrated, and cleared. Then, the tissue was added dropwise with neutral resin and treated overnight at room temperature. On the next day, images were collected and analyzed under a microscope.

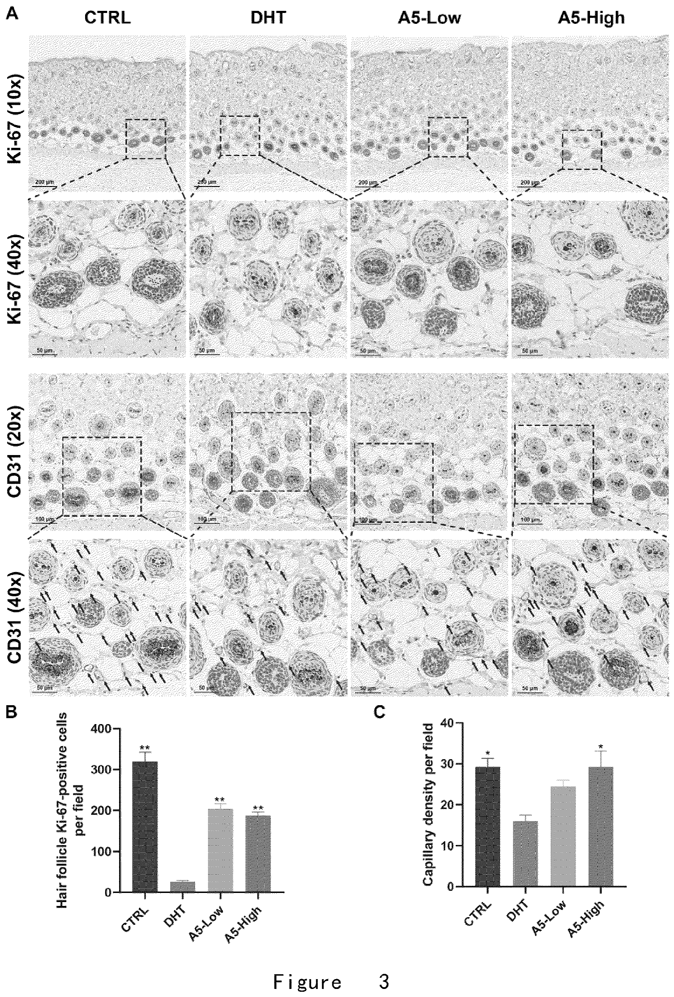

Results: As shown in Figure 3, immunohistochemical staining was used to detect Ki67 for evaluating cell proliferation in hair follicle cells, and to detect CD31 for evaluating the number of capillaries around the dermal papillae. After continuous injection of DHT, compared with the control group, the proliferation ability of cells in the hair follicles of mice in androgenetic alopecia model was significantly reduced, and the number of capillaries around the dermal papillae was significantly reduced. However, after receiving treatment with Annexin A5, the proliferation and survival ability of cells in the hair follicles were improved, and the number of capillaries increased,

### Example 4: Analysis of androgen receptor and TGF-β1 expression in hair follicle specimens

Sample Treatment: After euthanizing the mice, 1 cm² skin tissue was collected, and the hair on the skin surface was removed with a scissors as much as possible. The dermal papillae were seperated from the skin, and collected in an ice box, lysis buffer was added, and homogenization and sample lysis were performed. The supernatant was collected by high-speed centrifugation, the protein concentration was determined using BCA method, and protein samples were prepared according to Western Blot requirements.

Western Blot detection of protein expression levels: SDS polyacrylamide separation gel was prepared according to the molecular weight of the protein. After gel polymerization, electrophoresis buffer was added, and the comb was removed to load the sample in the predetermined order. The protein sample was thoroughly separated by running the gel under constant voltage according to molecular weight (the specific running time can be adjusted according to the actual situation). After electrophoresis, the gel was carefully taken off, the transfer clamp was prepared, and placed in the transfer cassette for transfer under the condition of 200 mA for 2 hours (which can be adjusted according to the protein molecular weight). An ice-water mixture should be added to the outside of the transfer tank to achieve the effect of cooling and avoid excessive temperature that may affect transfer efficiency. After the membrane transfer was completed, the PVDF membrane was taken off, blocked in 5% milk, and shaken gently on a shaker at room temperature for 2 hours. Subsequently, the PVDF membrane was cut and placed in the corresponding primary antibody, and incubated overnight at 4 °C. On the next day, the membrane was washed three times with 1 × TBST for 5 minutes each time, and then incubated with the secondary antibody at room temperature for 2 hours before washing the membrane three times for 5 minutes each time. Fresh developing solution was prepared. The membrane was exposed using a chemiluminescence imager to develop the film. The exposure time should be adjusted according to different antibodies used, and the gel images were saved after completion.

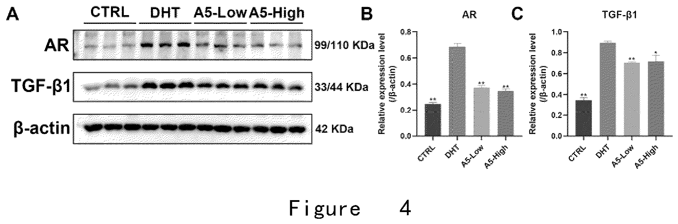

Results: As shown in Figure 4, the dermal papillae of mice were isolated and analyzed using Western Blot assay to detect the expression levels of androgen receptor (AR) and transforming growth factor β1 (TGF β-1). The androgen receptor and TGF-β1 were expressed in normal hair follicles (CTRL group), and after treatment with DHT, the expression of AR and TGF-β1 in mice was significantly increased (DHT group). Mice treated with Annexin A5 after modeling (A5-Low/A5 High group) showed significant inhibition of AR and TGF-β1 expression in their bodies.

Experiments were carried out according to the above scheme, the experimental results obtained can effectively prove that Annexin A5 can promote hair regeneration in mice with androgenetic alopecia. Specifically, by constructing a mouse model of androgenetic alopecia and treating it with Annexin A5, the skin of the experimental group mice changed from pink to black earlier after depilation, indicating that Annexin A5 treatment can help mice enter the growth phase of hair, promote hair growth, and increase hair coverage. By observing the number of mouse hair follicles through histological HE staining, it was found that modeling or drug treatment did not affect the number of dermal papillae in hair follicle. Ki67 immunohistochemistry assay was used to evaluate the proliferation of cells in hair follicles, and found that after continuous injection of DHT, the proliferation ability of cells in hair follicles was significantly reduced. After treatment with Annexin A5, the proliferation level of cells in hair follicles was upregulated. CD31 immunohistochemical staining was used to evaluate the number of capillaries around the dermal papillae, and found that after continuous injection of DHT, the number of capillaries around the dermal papillae in mice decreased significantly, while the number of capillaries increased after treatment with Annexin A5. By isolating the dermal papillae of mice, it was found that after treatment with Annexin A5, the expression of androgen receptors and transforming growth factor β1 in the body was downregulated compared to the control group, thereby promoting hair growth. Therefore, Annexin A5 is an effective ingredient for treating hair loss and improving hair growth quality, and can be used as an effective method for treating hair loss.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. Use of Annexin A5 in the preparation of a pharmaceutical composition or a medical device for treating hair loss.

2. The use according to claim 1, **characterized in that** the hair loss is selected from the group consisting of: androgenetic alopecia, alopecia areata, Telogen effluvium, senescent alopecia, psychogenic alopecia, physical alopecia, chemical alopecia, nutritional alopecia, infectious alopecia, and endocrine alopecia.

3. The use according to claim 1, **characterized in that** the pharmaceutical composition is used for (1) promoting hair growth; (2) regenerating hair after hair loss; (3) improving the proliferation ability of cells in hair follicles; (4) increasing the number of capillaries around the dermal papilla; and (5) inhibiting the expression of androgen receptor (AR) and transforming growth factor β1 (TGF-β1) in hair follicles.

4. Use of Annexin A5 in the preparation of products for treating hair growth and/or preventing hair loss.

5. The use according to claim 4, **characterized in that** the product is used for (a) improving the smoothness of hair; (b) reducing hair loss; (c) increasing hair growth density; and/or (d) promoting hair growth.

6. The use according to claim 4, **characterized in that** the product is used for hair nurturing and/or hair care.

7. A pharmaceutical composition for treating hair loss, the pharmaceutical composition comprises: (i) Annexin A5; (ii) other drugs for treating hair loss; and (iii) pharmaceutically acceptable carriers.

8. The pharmaceutical composition according to claim 7, **characterized in that** the other drugs are selected from the group consisting of Qingwanye, minoxidil, and finasteride.

9. The pharmaceutical composition according to claim 7, **characterized in that** the pharmaceutical composition is introduced into the body tissue by a method selected from the group consisting of: injection, spray, permeation, absorption, physically mediated method, and chemically mediated method, or introduced into the body tissue after mixing with or being encapsulated by other substances.

10. The pharmaceutical composition according to claim 7, **characterized in that** the dosage form of the pharmaceutical composition is selected from the group consisting of: liniments, sprays, lotions, ointments, and injections.
